# EUROPÄISCHE PATENTSCHRIFT

(11) **EP 0 539 654 B1**
(45) Veröffentlichungstag und Bekanntmachung des Hinweises auf die Patenterteilung: **03.09.1997**
(21) Anmeldenummer: 92109446.2
(22) Anmeldetag: 04.06.1992
(51) Int. Cl.: A61F 2/38

(54) **Kniegelenk-Endoprothesensystem**
System of a knee joint endoprosthesis
Système d'une endoprothèse du genou

(30) Priorität: 11.06.1991 DE 4119226
(43) Veröffentlichungstag der Anmeldung: 05.05.1993
(73) Patentinhaber: GMT Gesellschaft für medizinische Technik mbH, D-22767 Hamburg (DE)
(72) Erfinder: Nieder, Elmar Dr., W-2155 York (DE)
(74) Vertreter: Heldt, Gert, Dr. Dipl.-Ing.

(56) Entgegenhaltungen:
- EP-A- 0 488 378
- DE-A- 3 119 841
- US-A- 3 813 700
- US-A- 4 938 769

## Beschreibung

Die Erfindung betrifft ein Kniegelenk-Endoprothesensystem mit einem Femurteil und einem Tibiateil, die um einen Scharnierbolzen eines sie miteinander verbindenden Scharniergelenkes von einer gestreckten Stellung in eine abgewinkelte Stellung verschwenkbar gelagert sind und jeweils Schäfte aufweisen, die in abgewinkelter Stellung einen Beugewinkel Zwischen sich einschließen, in gestreckter Stellung in einander entgegengesetzte Richtungen weisen und von denen ein Tibiaschaft an seinem dem Femurteil Zugewandten oberen Ende in ein Tibiaplateau und ein Femurschaft mit seinem dem Tibiateil zugewandten unteren Ende in ein Gehäuse einmündet, in dessen Wandungen der Schanierbolzen gelagert ist, und an deren dem Tibiateil Zugewandten unteren Enden jeweils eine Kufe befestigt ist.

Eine derartige Kniegelenkendoprothese ist aus der DE-A-3119841 bekannt. Sie hat sich in der Vergangenheit gut bewährt, um Patienten die Gehfähigkeit zu erhalten bzw. zu verbessern. Sie kann jedoch in einer Vielzahl von Fällen, die im einzelnen erläutert werden sollen, nicht zum Einsatz kommen. Grundsätzlich stehen für die Versorgung arthrotisch entzündlich oder traumatisch veränderter Kniegelenke zwei voneinander verschiedene Arten von Ersatzmöglichkeiten zur Verfügung, der Oberflächenersatz und der Totalersatz.

Der Oberflächenersatz ersetzt die Oberflächen der Femurkondylen und Tibiakondylen und arbeitet mit den Bändern (mediales Seitenband, laterales Seitenband, Kreuzbänder) zusammen. Der Oberflächenersatz kooperiert mit den Bändern in der Lastübetragung und der Steuerung des Bewegungsablaufes. Damit der Oberflächenersatz gut funktioniert, müssen die vier Bänder weitgehend intakt sein, d. h. die Kniegelenkerkrankung (genu varum, genu valgum, genu flexum, genu laxum) darf nicht nicht kompensiert sein. Dekompensation bedeutet Schrumpfung oder Überdehnung der passiven und aktiven Stabilisatoren des Kniegelenkes oder einer Kombination von beidem. Die passiven Stabilisatoren des Kniegelenkes sind die erwähnten Bänder. Die aktiven Stabilisatoren sind die Sehen der Muskeln,die das Kniegelenk überbrücken. Sie lassen sich unterteilen in die medialen Stabilisatoren, die lateralen Stabilisatoren und dorsalen Stabilisatoren, wenn vom Streckapparat abgesehen wird. Gute Funktion und Langlebigkeit eines Oberflächenersatzes ist dann nicht mehr garantiert, wenn die passiven und aktiven Stabilisatoren dekompensiert sind (geschrumpft oder überdehnt), da es sich bei dem Oberflächenersatz um völlig ungekoppelte Systeme handelt. Oberflächenersatz mit ebenen Plateaus (Schlittenprothese) bedürfen vollkommen intakter Stabilisatoren. Der Oberflächenersatz mit leicht gemuldeten Plateaus kompensiert bis zu einem gewissen Grade leichtere Schwächungen der Stabilisatoren.

Sind die passiven und aktiven Stabilisatoren dekompensiert, so ist der Totalersatz erforderlich, um die entsprechenden Schwächungen zu kompensieren. Beim Totalersatz werden die mechanischen Funktionen, d. h. die Lastübetragung und die Steuerung des Bewegungsablaufes von der prothetischen Konstruktion total übernommen. Alle Formen des Totalersatzes haben weniger Freiheitsgrade der Bewegung als die Formen des Oberflächenersatzes. Dafür erzeugen sie im Falle nicht intakter Stabilisatoren umso mehr Stabilität im Kniegelenk.

Die extreme Form des Totalersatzes ist eine reine Scharnierprothese mit nur einem Freiheitsgrad der Bewegung, der monozentrischen Beugung. Demgegenüber weist die Rotationsprothese über den Freiheitsgrad der monozentrischen Beugung noch den Freiheitsgrad der tibialen Rotation auf. Die tibiale Rotation dient dem Schutz der Verankerung und des Knochens von Oberschenkel und Unterschenkel, da Energie aus Momenten, die durch Drehungen um die Längsachse des Beines entstehen, in die restlichen Weichteilstrukturen eingeleitet werden, die um das Knie verblieben sind. Im klinischen Einsatz ist aus diesem Grunde die Haltbarkeit der Verankerung der Rotationsprothese länger als die der Scharnierprothese. Darüber hinaus ist die Möglichkeit zur Ober- oder Unterschenkelfraktur nach Trauma geringer. Da nachweislich Verankerung und Knochen bei der Rotationsprothese stärker geschützt werden, wird die Rotationsprothese gegenüber der Scharnierprothese favorisiert. Allerdings gibt es Indikationsbeschränkungen für die Rotationsprothese, und diese Einschränkungen sind bei den unterschiedlichen Deformitäten des Kniegelenkes (genu varum, genu valgum, genu flexum, genu laxum) unterschiedlich.

### genu flexum

Das genu flexum ist eine Deformität bei dem das Kniegelenk nicht voll gestreckt werden kann. Der Grund für diese Beugefehlstellung ist eine Schrumpfung der hinteren Kapsel und/oder eine Schrumpfung der Beugesehnen Zur Beseitigung solch einer Beugekontraktur wird im Rahmen der normalen operativen Technik die hintere Kapsel an ihrem Ansatz am dorsalen Oberschenkel abgelöst. Bei den meisten Beugekontrakturen reicht dies aus, um das Kniegelenk nach Implantation der Rotationsprothese in die volle Streckung zu bringen. Ist das genu flexum sehr ausgeprägt, liegt darüber hinaus meistens eine Schrumpfung der Beugesehnen vor. Um solche Kniegelenke aufzurichten, gibt es zwei Möglichkeiten. Entweder wird eine Verlängerungsplastik (Z-Plastik) der Beugesehnen durchgeführt oder aber es wird schrittweise vermehrt tibial resiziert. Verlängerungsplastiken der Beugesehnen sind aufwendig, erfordern Zusatzeingriffe und die post operativ notwendige Immobilisierung des Patienten widerspricht dem Prinzip der Frühmobilisierung nach Kniegelenkersatz. Aus diesem Grunde wird eine schrittweise tibiale Resektion bis zur vollen Streckbarkeit des Kniegelenkes favorisiert. Ist zur Erreichung der vollen Streckung eine forcierte tibiale Resektion erforderlich, so entsteht lediglich Pseudostabilität in Streckstellung durch die Anspannung der erhaltenen dorsalen verkürzten Strecksehnen, jedoch in Beugestellung sind alle das Kniegelenk überbrückenden Strukturen, der Streckapparat und die verbliebene Kapsel-Bandmanschette bzw. deren narbiges Regenerat relativ zu lang und es droht die Gefahr der funktions- und materialgefährdenden Subluxation oder sogar der Luxation im Gelenk zwischen Zapfen und Hülse des Rotationsknies. Aus diesem Grunde wird bei Erfordernis der forcierten tibialen Resektion in diesen Fällen das Scharniergelenk implantiert, bei dem die femurale und tibiale Komponente durch den Achsmechanismus gekoppelt sind.

### genu varum

Das genu varum ist meist und lange lediglich gekennzeichnet durch einen Niveauverlust des medialen Femur- und des medialen Tibiakondylus. Die passiven und aktiven medialen und lateralen Stabilisatoren dekompensieren nur selten und spät, d. h. es kommt nur selten und nur bei sehr starker Ausprägung des O-Beines zu einer Schrumpfung der medialen Strukturen und einer Überdehnung der lateralen Strukturen. Der Spielraum für die Rotationsprothese ist beim genu varum fast unbegrenzt, da die geschrumpften und überdehnten Bänder im Rahmen der normalen operativen Technik ohnehin abgelöst werden und das narbige Regenerat aus Seitenbändern und Kapsel in der Regel ausreicht, um den engen Kontakt zwischen der femuralen und der tibialen Komponente des Rotationsknies zu garantieren. Die Scharnierprothese ist beim genu varum nur dann indiziert, wenn das genu varum mit einem ausgeprägten genu flexum kombiniert ist, und zwar dann, wenn die beschriebene forcierte tibiale Resektion zur Korrektur der Flexionskomponente erforderlich ist.

### genu valgum (X-Bein)

Beim genu valgum dekompensieren die medialen und lateralen Stabilisatoren sehr frühzeitig. Dies allein begrenzt den Einsatz der Rotationsprothese nicht, weil wie beim genu varum ohnehin die Seitenbänder am Femurkondylus gelöst werden und die narbige Kapselseitenbandregeneratmanschette den innigen Kontakt der Komponenten garantiert. Beim genu valgum gibt es jedoch komplizierende Zusatzfaktoren. Auf der Außenseite des Kniegelenkes dominieren die aktiven Stabilisatoren (muskulus bizeps und seine Sehne, traktus iliotibialis, lateraler Gastoknemiuskopf, muskulus poblitius und seine Sehne). Diese aktiven Stabilisatoren der Außenseite des Kniegelenkes rotieren in Beugestellung die tibia nach außen. Dies ist ihre normale Zusatzfunktion. Sind diese aktiven Stabilisatoren bzw. deren Sehnen im Rahmen der frühzeitigen Dekompensation bei genu valgum jedoch geschrumpft, so wird das genu valgum durch eine Außenrotationsfehlstellung des Unterschenkels kompliziert. Um dem zu begegnen, müßte man die aktiven Stabilisatoren bzw. deren Sehnen durch Z-Plastiken verlängern. Dies ist wie bei den Beugesehnen bei genu flexum aufwendig und erfordert wiederum postoperative Immobilisierung. Aus diesem Grunde werden beim genu valgum mit Außenrotationsfehlstellung diese aktiven außendrehenden geschrumpften Stabilisatoren gekerbt oder sogar abgetrennt und die dadurch erzeugte Schwächung oder eine noch verbliebene Neigung zur Außenrotationsfehlstellung wird durch eine Scharnierprothese kompensiert.

### genu laxum

Das genu laxum ist gekennzeichnet durch einen extreme Überdehnung sämtlicher Stabilisatoren. Daher gibt es bei genu laxum jeglicher Genese keine Indikation mehr für die Rotationsprothese. Es muß immer die Scharnierprothese verwendet werden, um die tibiale und femurale Komponente zusammenzuhalten.

Diese Einschränkungen bei der Verwendung der Rotationsprothese gelten im wesentlichen für die häufigsten Erkrankungen, nämlich die idiopartische Arthrose. Bei rheumatischer Arthritis, der nächsthäufigsten Erkrankung, die als entzündliche Erkrankung zum Kniegelenkersatz führt, gelten diese Bedingungen verschärft; denn die rheumatischen Patienten leiden unter einer allgemeinen Muskelschwäche, so daß die aktiven Stabilisatoren ohnehin geschwächt sind. Außerdem ist die rheumatische Arthritis im wesentlichen durch ein genu valgum mit Außenrotationsfehlstellung gekennzeichnet, so daß bei rheumaticher Arthritis aus diesem Grunde die Scharnierprothese gegenüber der Rotationsprothese oft favorisiert wird.

Diese gerade in letzter Zeit verbesserte Kenntnis von Kniegelenkerkrankungen hat deutlich gemacht, daß selbst intensive Voruntersuchungen auch mit Hilfe der Röntgentechnik sehr häufig nicht ausreicht, um die Art der zu verwendenden Prothese im einzelnen vorplanen zu können. Insbesondere hängt die Auswahl des zu implantierenden Ersatzes sehr häufig von Befunden ab, die der operierende Arzt erst nach der operativen Öffnung des Kniegelenkes treffen kann. So kann er beispielsweise im genu flexum sehr häufig erst nach der Lösung der hinteren Kapsel entscheiden, wie viel Knochenmasse er tibial resizieren muß. Andererseits hängt seine Entscheidung, welche der zur Verfügung stehenden Prothesen in einem konkreten Fall zu implantieren ist, in starkem Umfange davon ab, wie viel Knochenmasse tibial resiziert werden mußte.

Im Hinblick auf die häufig erst nach der operativen Öffnung des Kniegelenkes zur Verfügung stehenden Entscheidungsgrundlagen, welche Kniegelenk-Endoprothese zweckmäßigerweise zum Einsatz kommen muß, war bisher eine Vorplanung einer Kniegelenkoperation so gut wie ausgeschlossen. Hatte sich der operierende Arzt beispielsweise darauf eingestellt, daß der Patient mit einer Rotations-Endoprothese versorgt werden sollte, so stellte sich häufig erst beim Resizieren der Knochenmasse heraus, daß nicht eine Rotations-Endoprothese sondern eine Scharniergelenk-Prothese zum Einsatz kommen mußte. In diesem Falle mußte möglicherweise die Planung der Operation neu überdacht werden. Mit einer erheblichen Verzögerung des Operationsablaufes war zu rechnen, da dem Arzt für die Durchführung der Operation lediglich ein Tibiateil und ein Femurteil zum Einsatz in den jeweiligen Knochen zur Verfügung stand.

Aufgabe der vorliegenden Erfindung ist es daher, das Kniegelenk-Endoprothesensystem der einleitend genannten Art so zu verbessern, daß es in einer Vielzahl unterschiedlich gelagerter Bedarfsfälle Verwendung finden kann.

Diese Aufgabe wird erfindungsgemäß dadurch gelöst, daß das Kniegelenk-Endoprothesensystem aus einem Rotationstibiateil, einem Scharniertibiateil und einem Femurteil besteht, das einerseits jeweils um eine senkrecht auf dem Tibiaplateau stehende Rotationsachse rotationsbeweglich gegenüber dem zugeordneten Rotationstibiateil und andererseits rotationsunbeweglich in einer fest mit einem Scharniertibiateil verbundenen Halterung gelagert ist und die Tibiateile untereinander und das Femurteil mit jeweils einem Tibiateil gleiche äußere Abmessungen aufweisen.

Mit einer derartigen Kniegelenk-Endoprothese wird der operierende Arzt in den Stand versetzt, eine Entscheidung darüber, welche Paarung eines Femurteils mit einem Tibiateil in einer konkreten Operationssituation zu verwenden ist, erst treffen müssen, wenn er sämtliche Faktoren für eine derartige Entscheidung genau übersehen kann. Die operative Technik für beide Prothesen ist gleich, weil die äußere Form beider Prothesen identisch ist. Die Operation kann von der Beendigung der Weichteilschnitte bis zur Anpassung der Ober- und Unterschenkelkondylen zur Aufnahme des Kniegelenkes durchgeführt werden. Im Rahmen einer Probereposition, d. h. einem Einsetzen der Prothese in den Knochen ohne Zement kann genau entschieden werden, ob eine Rotationsprothese oder eine Scharnierprothese verwendet werden soll, denn die beschriebenen limitierenden Faktoren für die auszuwählende Rotationsprothese müssen erst genau erkennbar sein, bevor die ausgewählte Prothese fest eingesetzt wird.

Der Vielzahl der zu treffenden Auswahlentscheidungen wurde bisher dadurch Rechnung getragen, daß Prothesen für den Kniegelenkersatz verwendet wurden, deren Funktion eine eindeutige Aussage darüber nicht zuließ, in welchen Einsatzfällen sie implantiert werden konnte, und welche Einsatzfälle von ihr nicht mehr abgedeckt werden konnten. So sind beispielsweise Knieendoprothesen bekanntgeworden, deren Schwenkachsen durch eine nicht eindeutige Lagerung ein gewisses, aber nicht unbegrenztes Spiel eingeräumt wurde. Alle diese Endoprothesen besitzen den Nachteil, daß sie weder für den Einsatzfall streng geführter Beugebewegungen noch für den Fall einer zulässigen tibialen Rotation ausreichend geeignet sind. Sie führen sehr häufig zu Fehlfunktionen und einem sehr schnellen Verschleiß.

Demgegenüber besitzt das erfindungsgemäße Endoprothesensystem den Vorteil einer optimalen Eignung für den jeweils diagnostizierten Einsatzfall, ohne daß deswegen auf eine optimale Funktion der jeweilig eingesetzten Prothese verzichtet werden müßte. Darüber hinaus kann die konkrete Auswahl der richten Prothesenvariante nach probeweisem Einsetzen des Systems getroffen werden. Der Operateur trifft seine Entscheidung, wenn alle zur Korrektur der jeweiligen Deformität (genu varum, genu valgum, genu flexum, genu laxum) notwendigen Operationsschritte durchgeführt worden sind und ihm somit alle Auswahlparameter bekannt sind, so daß danach keine Notwendigkeit mehr auftreten kann, wegen der Wahl eines anderen Prothesentyps z. B. noch andere Knochenresektionsschnitte vorzunehmen, welche zu äußerst nachteiligen zusätzlichen Knochenverlusten führen würden.

Obgleich die einschlägige Fachwelt diesem Lösungsansatz skeptisch gegenüberstand, weil sie der Auffassung war, daß eine Rotationsprothese im Hinblick auf die von ihr erwartete Funktion eine andere Ausbildung besitzen müsse als die Scharnierprothese, haben Versuche klargestellt, daß die beiden Prothesenarten als Teile eines Systems ausgebildet werden können, das gleiche Abmessungen aufweist. Darüber hinaus hat sich ergeben, daß diese gleiche Ausbildung weder zu einer Über- noch zu einer Unterdimensionierung der einen oder der anderen Prothesenart führt.

Gemäß einer bevorzugten Ausführungsform der Erfindung ist der Tibiaschaft sowohl in einer den Beugewinkel einschließenden Beugeebene als auch in einer senkrecht zu dieser verlaufenden Querebene senkrecht zu einer ihm zugewandten Unterseite des Tibiaplateaus befestigt. Auf diese Weise ist gewährleistet, daß unabhängig von der jeweiligen Verwendung des Tibiateils die auftretenden Kräfte vom Tibiaplateau über den Tibiaschaft optimal in den Unterschenkelknochen eingeleitet werden können.

Gemäß einer weiteren bevorzugten Ausführungsform der Erfindung ist auf dem Tibiaplateau ein Gleitaufsatz befestigt, dem auf seinen beiden einander bezüglich einer sich durch den Tibiaschaft planparallel zur Beugeebene erstreckenden Mittelebene gegenüberliegenden Seitenteilen Gleitflächen der Kufen zugewandt sind. Dieser Gleitaufsatz ermöglicht eine optimale Übertragung der im Femurteil auftretenden Kräfte auf den Tibiateil, unabhängig davon, ob der Femurteil gegenüber dem Tibiateil rotationsbeweglich gelagert ist oder ausschließlich Schwenkbewegungen ausführen kann.

Gemäß einer weiteren bevorzugten Ausführungsform der Erfindung ist der Femurteil in gestreckter Stellung der Endoprothese mit einer bogenförmig zwischen zwei Gleitflächen der beiden Kufen verlaufenden Unterkante auf einem Kamm geführt, der auf dem Gleitaufsatz ausgebildet ist. Auf diese Weise wird sowohl die Rotationsprothese als auch die Scharnierprothese in gestreckter Stellung eindeutig stabilisiert, so daß das Schwenkgelenk weitgehend von Rotationsmomenten entlastet ist.

Weitere Einzelheiten der Erfindung ergeben sich aus der nachfolgenden ausführlichen Beschreibung und den beigefügten Zeichnungen, in denen eine bevorzugte Ausführungsform der Erfindung beispielsweise veranschaulicht ist.

### In den Zeichnungen zeigen:

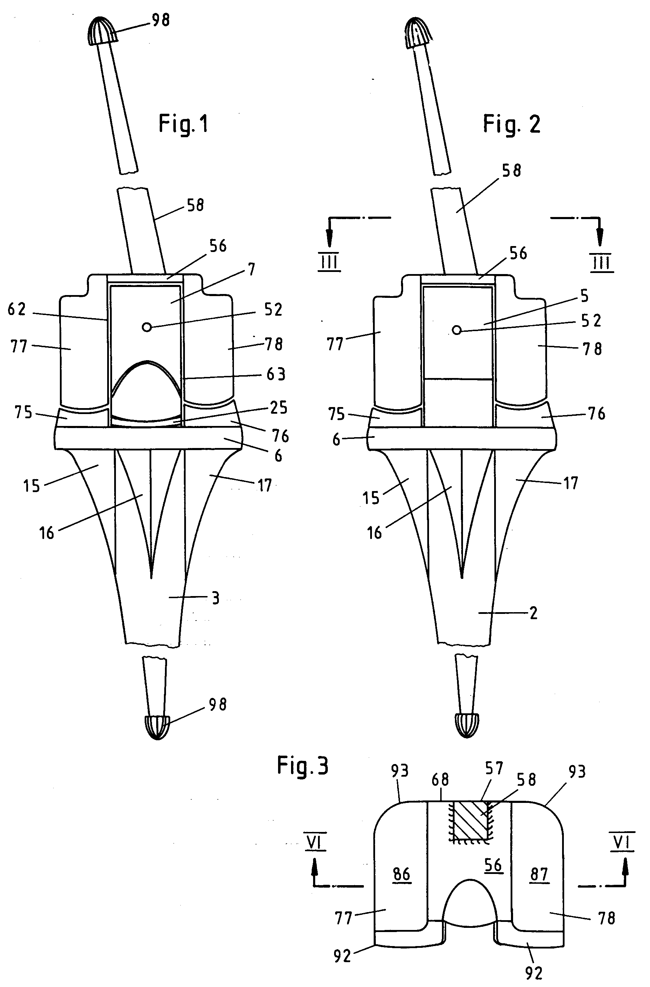

- Fig. 1: eine Ansicht auf ein Hinterteil einer Rotationsknieendoprothese,
- Fig. 2: eine Ansicht auf den Hinterteil einer Scharnierknieendoprothese,
- Fig 3: eine teilweise geschnittene Draufsicht auf einen Femurteil,

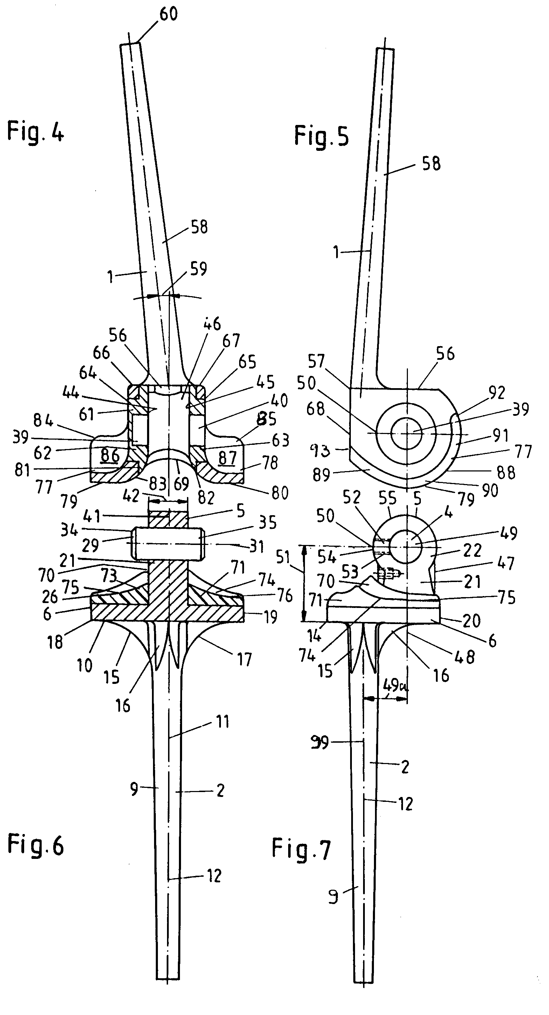

- Fig. 4: eine teilweise geschnittene Ansicht eines Femurteils entsprechend der Schnittlinie IV-IV in Fig. 3,
- Fig. 5: eine Seitenansicht eines Femurteils,
- Fig. 6: eine teilweise geschnittene Ansicht des Tibiateils einer Scharnierknieendoprothese, bei der der Schnitt in derselben Schnittebene verläuft wie Fig. 4,
- Fig. 7: eine Seitenansicht eines Tibiateils einer Scharnierknieendoprothese,

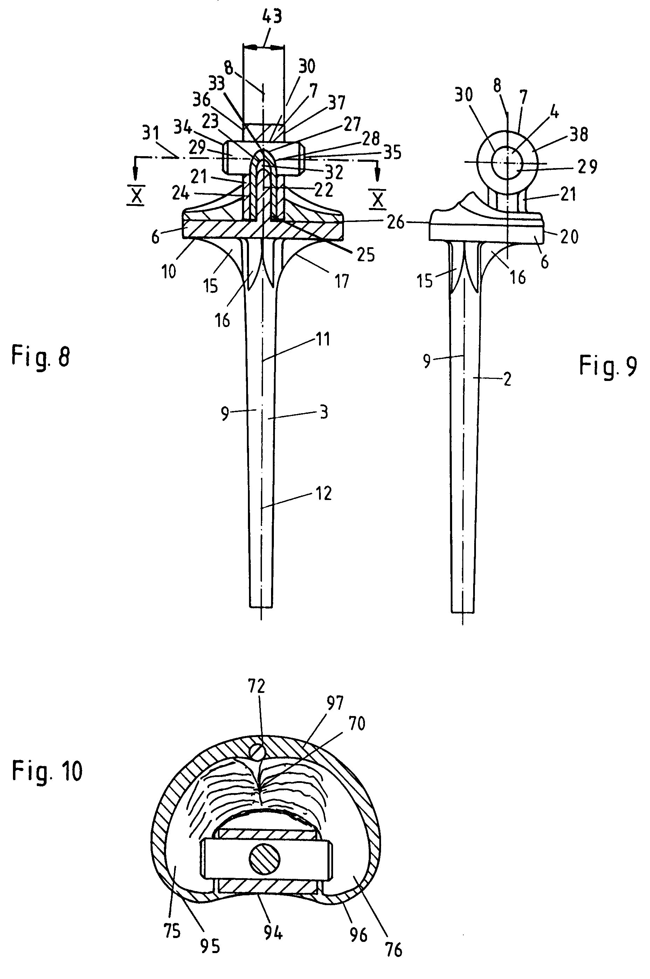

- Fig. 8: eine teilweise geschnittene Ansicht eines Tibiateils einer Rotationsknieendoprothese, bei der der Schnitt in derselben Schnittebene verläuft wie in Fig. 4,
- Fig. 9: eine Seitenansicht eines Tibiateils einer Rotationsknieendoprothese,
- Fig. 10: eine teilweise geschnittene Draufsicht auf den Tibiateil einer Rotationsknieendoprothese entsprechend der Schnittlinie X-X in Fig. 8,
- Fig. 11: eine schematische Darstellung einer Endoprothese in gestreckter Stellung und
- Fig.12: eine schematische Darstellung einer Endoprothese in gebeugter Stellung.

Ein Endoprothesensystem für ein Kniegelenk besteht im wesentlichen aus einem Femurteil 1, dem je nach dem jeweils während einer Operation auftretenden Bedarf ein Scharniertibiateil 2 oder ein Rotationstibiateil 3 zugeordnet werden kann. Die Verbindung des Femurteils 1 mit jeweils einem der beiden Tibiateile 2, 3 erfolgt mit Hilfe eines Schwenkgelenkes 4, das im Falle des Scharniertibiateils 2 über einen Steg 5 fest mit einem Tibiaplateau 6 verbunden ist, während es im Falle des Rotationstibiateils 3 in einem Rotationskörper 7 um eine senkrecht auf dem Tibiaplateau 6 stehende Rotationsachse 8 verschwenkbar gelagert ist.

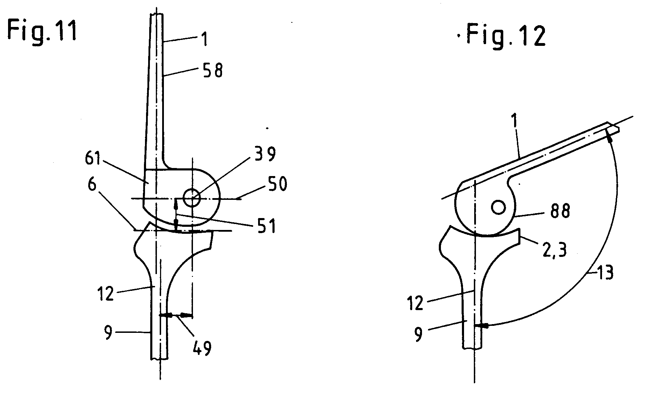

Die beiden Tibiateile 2, 3 besitzen jeweils einen Tibiaschaft 9, der senkrecht auf einer dem Schwenkgelenk 4 abgewandten Unterseite 10 des Tibiaplateaus 6 befestigt ist. Dabei verläuft der Tibiaschaft 9 in einer Beugeebene 11, die sich zentrisch durch das Tibiaplateau 6 erstreckt und von einer sich durch den Tibiaschaft 9 erstreckenden Mittellinie 12 aufgespannt wird, wenn der Tibiateil 2, 3 um einen Beugewinkel 13 gegenüber dem Femurteil 1 verschwenkt wird (Fig. 12).

Dabei ist der Tibiaschaft 9 in einem vom Beugewinkel 13 abgewandten vorderen Teil 14 des Tibiaplateaus befestigt. Er ist mit Hilfe von drei Rippen 15, 16, 17 gegenüber der Unterseite der Tibiaplateaus 6 abgestützt. Die beiden Rippen 15, 17 erstrecken sich quer zur Beugeebene 11 unter zwei sich einander gegenüberliegenden Seitenteile 18, 19 des Tibiaplateaus, während die Rippe 16 sich in Richtung der Beugeebene 11 zu einem vom Beugewinkel 13 eingeschlossenen Hinterteil 20 des Tibiaplateaus 6 erstreckt.

Auf dem Hinterteil 20 des Tibiaplateaus 6 erhebt sich sowohl beim Rotationstibiateil 3 als auch beim Scharniertibiateil 2 eine Halterung 21, in der das Schwenkgelenk 4 befestigt ist. Diese Halterung 21 ist je nach der Verwendung der Knieendoprothese als Steg 5 fest mit dem Tibiaplateau 6 verbunden oder als Rotationskörper 7 um die Rotationsachse 8 schwenkbar auf einem Stift 22 gelagert, der fest mit dem Tibiaplateau 6 verbunden ist und in Richtung der Rotationsachse 8 verläuft. Auf diesem Stift 22 gleitet der Rotationskörper 7 bei der Ausführung von Rotationsbewegungen um die Rotationsachse 8 auf einem Gleitlager 23, das aus einer Kunststoffhülse besteht. Diese Kunststoffhülse ist mit ihrer etwa zylindrischen Außenfläche in eine Bohrung 24 eingepaßt, die sich durch den Rotationskörper 7 zylindrisch erstreckt. Dabei ist die Kunststoffhülse mit einem Innendurchmesser versehen, der etwa dem Außendurchmesser des Stiftes 22 entspricht, so daß mit Hilfe der in die Bohrung 24 fest einpaßten Kunststoffhülse der Rotationskörper 7 gleitend auf dem Stift 22 gelagert ist. Die Einpassung der als Gleitlager 23 ausgebildeten Kunststoffhülse erfolgt in der Weise, daß sie mit ihrem dem Tibiaplateau 6 zugewandten unteren Ende 25 knapp oberhalb einer der Unterseite 10 abgewandten Oberseite 26 des Tibiaplateaus 6 endet, so daß bei der Ausführung von Rotationsbewegungen um die Rotationsachse 8 das untere Ende 25 die Oberseite 26 des Tibiaplateaus 6 nicht beaufschlagt.

Mit ihrem dem unteren Ende 25 abgewandten oberen Ende 27 ragt die als Gleitlager 23 ausgebildete Kunststoffhülse in eine entsprechend ausgebildete Ausnehmung 28 eines Bolzens 29 hinein, der sich durch einen im Rotationskörper 7 vorgesehene Bohrung 30 erstreckt. Dabei verläuft eine Längsachse 31 des Bolzens 29 quer zur Richtung der Rotationsachse 8 und der Beugeebene 11.

Die Ausnehmung 28 besitzt eine sich quer zur Längsachse 31 sich erstreckende, in Richtung der Rotationsachse 8 verlaufende Mittellinie 32. Sie endet in Form einer Kuppel 33, in die das Gleitlager 23 mit einer ähnlich geformten Kuppel hineinragt und in der sie gegen Verschieben in lotrechter Richtung gesichert ist. Der Bolzen 29 überragt mit seinen beiden Enden 34, 35 seitliche Begrenzungen 36, 37 des in seinem dem Tibiaplateau 6 zugewandten oberen Ende als Auge 38 ausgebildeten Rotationskörpers 7. Auf diesen beiden Ende 34, 35 ist der Femurteil 1 in entsprechend gestalteten Lagerschalen 39, 40 schwenkbar gelagert.

Auf dem Scharniertibiateil 2 erhebt sich statt des Stiftes 22 der Steg 5, der ähnlich wie der Stift 22 fest mit dem Tibiaplateau 6 verbunden ist. Der Steg 5 steht senkrecht auf dem Tibiaplateau 6. Seine Mittelachse 41 verläuft in der Beugeebene 11. Quer zur Beugeebene 11 besitzt der Steg 5 eine Breite 42, die der Breite 43 des Rotationskörper 7 entspricht. Diese Breite 42, 43 ist so bemessen, daß die Halterung 21 in einem von ebenen Seitenwandungen 44, 45 begrenzten Innenraum 46 genau geführt wird, wenn jeweils Schwenkbewegungen um die Längsachse 31 des Bolzens 29 durchgeführt werden.

Der Steg 5 ist wie der Stift 22 auf dem Hinterteil 20 des Tibiaplateaus 6 angeordnet. Er schließt mit seiner Hinterkante 47 mit dem Hinterteil 20 des Tibiaplateau 6 ab. Zwischen der Mittellinie 12, die sich durch den Scharnierteil 2 erstreckt, und einer sich durch den Bolzen 29 entlang seiner Längsachse 31 erstreckenden senkrechten Mittelebene 48 besteht beim Scharniertibiateil 2 ein ebenso großer Abstand 49a wie der entsprechende Abstand beim Rotationstibiateil. Durch den Steg 5 erstreckt sich eine Bohrung 49 senkrecht zur Beugeebene 11. In dieser Bohrung 49 ist der Bolzen 29 befestigt. Durch den Bolzen 29 und damit auch durch die Bohrung 49 erstreckt sich eine horizontale Mittelebene 50, deren Abstand 51 von der Unterseite 10 des Tibiaplateaus 6 beim Scharniertibiateil 2 ebenso groß ist wie beim Rotationstibiateil 3.

Außerdem ist auch der Bolzen 29 innerhalb der Bohrung 49 befestigt, und zwar mit einer Madenschraube 52, die sich durch eine mit einem entsprechenden Endgewinde 53 versehene Bohrung 54 erstreckt, die von einem äußeren Umfang 55 des Stegs 5 in Richtung auf die Bohrung 49 verläuft. Nach der Befestigung des Bolzens 29 in der Bohrung 49 trägt der Bolzen 29 auf seinen aus dem Steg 5 herausragenden Enden 34, 35 die im Femurteil 1 befestigten Lagerschalen 39, 40.

Unabhängig davon, ob der Femurteil 1 mit einem Scharniertibiateil 2 oder einem Rotationstibiateil 3 zusammenarbeitet, ist er in gleicher Weise ausgebildet. Der Innenraum 46 ist unabhängig davon, ob er den Steg 5 oder den Rotationskörper 7 aufzunehmen hat, mit den gleichen Maßen versehen. Auch die Lagerschalen 39, 40 gestatten wahlweise die Aufnahme des Bolzens 29, unabhängig davon, ob er im Steg 5 oder im Rotationskörper befestigt ist.

Dabei ergeben sich auch keine Unterschiede hinsichtlich der Lagerung des Bolzens 29 in den Lagerschalen 39, 40, die in den Seitenwandungen 44, 45 gelagert sind. Die Seitenwandungen 44, 45 sind über eine Deckplatte 56 mit einander verbunden, auf deren vorderer Abschlußkante 57 sich ein Femurschaft 58 erhebt. Dieser besitzt gegenüber der Beugeebene 11 eine Schrägstellung, die einem Winkel 59 entspricht, dessen Größe 3 bis 8° erreichen kann. Dieser Winkel 59 entspricht der Valgusstellung eines Oberschenkelknochens.

Der Femurschaft 58 verjüngt sich in Richtung auf sein der Deckseite 56 abgewandtes Ende 60 konisch. Dabei kann der Querschnitt des Femurschaftes 58 rund mit abgeflachten Seitenkanten sein. Es ist jedoch auch möglich, daß der Femurschaft einen rechteckigen Querschnitt aufweist, dessen größere Kante sich quer zur Beugeebene erstreckt. In diesem Falle besitzt der Femurschaft 58 abgerundete Ecken. Er mündet mit Hohlkehlen in die Deckplatte 56 ein.

Der Innenraum 46 ist in einem Gehäuse 61 ausgebildet, das von der Deckplatte 56 nach oben und von Seitenwänden 62, 63 begrenzt wird, die sich planparallel zur Beugeebene 11 erstrecken. Auf den Seitenwänden 62, 63 sind die den Innenraum 46 begrenzenden Seitenwandungen 44, 45 ausgebildet. Planparallel dazu verlaufen Außenwandungen 64, 65, die ebenfalls planparallel zur Beugeebene 11 ausgerichtet sind. Die Seitenwände 62, 63 münden an ihren Oberkanten 66, 67 in die Deckplatte 56 ein, so daß ein quaderförmiges Gehäuse entsteht, das auf seiner der vorderen Abschlußkante 57 zugewandten Abschlußfläche 68 ebenflächig begrenzt ist. Die vordere Abschlußfläche 68 besitzt eine in Richtung auf den Innenraum 56 gewölbte Unterkante 69, in die bei gestreckter Stellung der Prothese ein Kamm 70 eingreift. Diese Kamm 70 ist auf einem Gleitaufsatz 71 ausgebildet, der das Tibiaplateau 6 auf seiner Oberseite 26 bedeckt. Der Gleitaufsatz 71 ist fest mit dem Tibiaplateau verbunden, und zwar mit Hilfe einer Befestigungsschraube 72, die im Bereich des Kammes 70 den Gleitaufsatz 71 mit dem Tibiaplateau 6 verschraubt. Durch das Eingreifen des Kammes 70 in die gewölbte Unterkante 69 erfolgt eine feste Führung des Femurteils 1 auf dem Tibiateil 2, 3, unabhängig davon, ob dieser als ein Scharniertibiateil 2 oder ein Rotationstibiateil 3 ausgebildet ist.

Beidseitig des Kammes 70 fällt der Gleitaufsatz 70 in sanften Hängen 73, 74 auf Gleitflächen 75, 76 ab, auf denen der Femurteil 1 mit Gleitkufen 77, 78 gleitet, wenn beispielsweise einseitige Belastungen eine Verformung im Bereich des Schwenkgelenkes 4 hervorrufen. Zu diesem Zwecke besitzen die Gleitkufen 77, 78 auf ihren dem Gleitaufsatz 71 zugewandten Unterseiten Gleitflächen 79, 80, die großflächig Kräfte aus dem Femurteil 1 auf eines der Tibiateile 2, 3 übertragen kann. Zu diesem Zweck sind die Gleitkufen 77, 78 an der Deckplatte 56 abgewandten Unterkanten 81, 82 der Seitenwände 62, 63 befestigt. Von den Unterkanten 81, 82 erstrecken sich die Gleitkufen 77, 78 in eine vom Innenraum 46 weg gewandte Richtung. Dabei sind die Gleitflächen 79, 80 in Form einer Außenwölbung 83 nach außen geführt und münden ein in ballig gestaltete horizontal nach außen geführte Flügel 84, 85, die auf ihren Oberseiten 86, 87 als Aufnahmeflächen für einen Femurknochen ausgebildet sind, in den der Femurteil 1 mit seinem Femurschaft 58 eingesetzt ist.

Die Gleitkufen 77, 78 erstrecken sich bogenförmig in die Lagerschalen 39, 40. Dabei bilden sie einen Bogen 88 aus, der in die vordere Abschlußfläche 68 des Gehäuses 61 einmündet. Von dort ausgehend verläuft er in einem relativ sanft geschwungenen vorderen Teil 89 und geht unmittelbar nach dem Durchlaufen der Mittelebene 48 in einen stärker gekrümmten Teil 90 über. Aus diesem verläuft er in Richtung auf die mittlere Horizontalebene 50 in einem schwächer gekrümmten hinteren Teil 91. Dabei beschreibt die sich aus den drei Teilen 89, 90, 91 zusammengesetzte gesamte Kufe 77, 78 einen Winkel von etwa 180°. Der Beginn des vorderen Teils liegt wenige Bogengrade unterhalb der mittleren Horizontalebene 50. Die Kufen 77, 78 enden an einem hinteren Ende 92, das etwa ebensoviele Bogengrade oberhalb der mittleren Horizontalebene 50 liegt wie der Beginn 93 unterhalb dieser Ebene 50 liegt.

Eine quer zur Beugeebene 11 verlaufende Querebene 99, die sich durch den Tibiaschaft 9 erstreckt, ist gegenüber einer das Tibiaplateau 6 in seinem vorderen Teil 14 begrenzenden Vorderkante in Richtung auf diese versetzt. Dabei ist die Querebene 99 um etwa ein Drittel einer sich in der Beugeebene 11 erstreckenden Längserstreckung der Unterseite 10 des Tibiaplateaus 6 versetzt.

Die durch den Bolzen 29 sich erstreckende Längsachse 31 verläuft in einem dem Hinterteil 20 zugewandten hinteren Drittel des Tibiaplateaus 6 quer zur Beugeebene 11. Durch den Bolzen 29 erstreckt sich eine Horizontalebene, die einen Abstand 51 von etwa 25 bis 30 mm vom Tibiaplateau 6 besitzt. Der Bolzen 29 hat einen Durchmesser von etwa 20 mm. Der Bogen 88, den die Gleitkufen 77, 78 um die Längsachse 31 des Bolzens 29 bilden, umgibt die Längsachse 31 in einem Abstand von etwa 20 bis 25 mm.

Die Abschlußfläche 68 ist im gestreckten Zustand der Endoprothese gegenüber dem vorderen Teil 14 in Richtung auf den Hinterteil 20 versetzt. Diese Versetzung macht etwa 10 bis 15 mm aus.

Die Gleitkufen 77, 78 überragen mit ihren hinteren Teil 91 den Hinterteil 20 des Tibiaplateaus 6. Der Abstand der hinteren Teile 91 gegenüber dem Hinterteil 20 des Tibiaplateaus 6 beträgt etwa 10 bis 15 mm.

Im gestreckten Zustand der Endoprothese verläuft die vordere Abschlußfläche 68 nicht planparallel zur Querebene 99. Vielmehr steht die vordere Abschlußfläche 68 im gestreckten Zustand der Endoprothese schräg auf dem Tibiaplateau 6, von dem sie sich schräg aufwärts in Richtung auf den Femurschaft 58 erstreckt. Sie mündet mit ihrer dem Tibiaplateau 6 zugewandten Unterkante 69 etwa in einem Bereich der Gleitfläche 75, 76 ein, in dem auf der Unterseite 10 des Tibiaplateaus 6 der Tibiaschaft 9 in das Tibiaplateau 6 einmündet.

In gestreckter Stellung der Prothese verläuft der Femurschaft in einer Querebene, die planparallel zur Querebene des Tibiaschaftes 9 verläuft. Die Querebene des Femurschaftes 58 ist in Richtung auf die Längsachse 31 gegenüber der Querebene des Tibiaschaftes 9 etwa 0,5 mm bis 4 mm versetzt.

Der Hinterteil 20 des Tibiaplateaus 6 weist in einem Mittelbereich eine sich in Richtung auf den vorderen Teil 14 erstreckende Einbuchtung 94 auf, die beidseits von zwei Ausbuchtungen 95, 96 begrenzt wird. Auf diesen Ausbuchtungen 95, 96 erstrecken sich von den Gleitkufen 77, 78 beaufschlagbare Gleitflächen 75, 76. Diese Gleitflächen 75, 76 sind im Bereich des vorderen Teils 14 hufeisenförmig durch einen den Kamm 70 ausbildenden Mittelteil 97 miteinander verbunden.

Das Schwenkgelenk 4 hält mit seiner Längsachse 31 einen Abstand von etwa 25 bis 30 mm vom Tibiaplateau 6. Das Gehäuse 61 weist zwischen seinen beiden Außenwandungen 64, 65 eine äußere Breite von etwa 30 bis 35 mm auf. Vorzugsweise ist das Gehäuse 30 mm breit. Das Gehäuse 61 besitzt Seitenwände 62, 63, die eine Dicke von etwa 5 bis 8 mm haben.

Sowohl der Femurschaft 58 als auch der Tibiaschaft 9 sind an ihren jeweiligen Enden 60 mit jeweils einem Zentrierstern 98 versehen, mit dessen Hilfe der jeweilige Schaft 58, 9 zielgenau in die ihm zugeordnete Knochenhöhle eingesetzt werden kann.

## Patentansprüche

1. Kniegelenk-Endoprothesensystem mit einem Femurteil (1) und einem Tibiateil(2, 3), die um einen Scharnierbolzen (29) eines sie miteinander verbindenden Scharniergelenkes (4) von einer gestreckten Stellung in eine abgewinkelte Stellung verschwenkbar gelagert sind, jeweils Schäfte (58, 9) aufweisen, die in abgewinkelter Stellung einen Beugewinkel (13) zwischen sich einschließen, in gestreckter Stellung in einander entgegengesetzte Richtungen weisen, von denen ein Tibiaschaft (9) an seinem dem Femurteil (1) zugewandten oberen Ende in ein Tibiaplateau (6) und ein Femurschaft (58) mit seinem dem Tibiateil (2, 3) zugewandten unteren Ende in ein Gehäuse (61) einmündet, in dessen Wandungen (62, 63) der Scharnierbolzen (29) gelagert ist und an deren dem Tibiateil (2, 3) zugewandten unteren Enden jeweils eine Kufe (77, 78) befestigt ist, dadurch gekennzeichnet, daß das Kniegelenk-Endoprothesensystem aus einem Rotationstibiateil (3), einem Scharniertibiateil (2) und einem Femurteil (1) besteht, das einerseits jeweils um eine senkrecht auf dem Tibiaplateau (6) stehende Rotationsachse (8) rotationsbeweglich gegenüber dem zugeordneten Rotationstibiateil (3) und andererseits rotationsunbeweglich in einer fest mit einem Scharniertibiateil (2) verbundenen Halterung (21) gelagert ist und die Tibiateile (2, 3) untereinander und das Femurteil (1) mit jeweils einem Tibialteil (2, 3) untereinander gleiche äußere Abmessungen aufweisen.

2. Kniegelenkendoprothese nach Anspruch 1, dadurch gekennzeichnet, daß der Tibiaschaft (9) in einer den Beugewinkel (13) einschließenden Beugeebene (11) senkrecht auf einer ihm zugewandten Unterseite (10) des Tibiaplateaus (6) befestigt ist.

3. Kniegelenkendoprothese nach Anspruch 1 oder 2, dadurch gekennzeichnet, daß der Tibiaschaft (9) auf der Unterseite (10) des Tibiaplateaus (6) mit drei Rippen (15, 16, 17) abgestützt ist, von denen zwei (15, 17) in einer durch den Tibiaschaft (9) verlaufenden, quer zur Beugeebene (11) ausgerichteten Querebene (99) und eine (16) in der Beugeebene (11) in Richtung auf einen vom Beugewinkel (13) eingeschlossenen Hinterteil (20) des Tibiaplateaus (6) verlaufen.

4. Kniegelenkendoprothese nach einem der Ansprüche 1 bis 3, dadurch gekennzeichnet, daß auf dem Tibiaplateau (6) ein Gleitaufsatz (71) befestigt ist, dem auf seinen beiden einander bezüglich einer sich durch den Tibiaschaft (9) planparallel zur Beugeebene (11) erstreckenden Mittelebene gegenüberliegenden Seitenteilen (18, 19) Gleitflächen (79, 80) der Gleitkufen (77, 78) zugewandt sind.

5. Kniegelenkendoprothese nach Anspruch 4, dadurch gekennzeichnet, daß die Seitenteile (18, 19) in Richtung auf einen Kamm (70) ansteigende Hänge (73, 74) aufweisen, die in einem dem Hinterteil (20) des Tibiaplateaus (6) gegenüberliegenden vorderen Teil (14) des Tibiaplateaus (6) angeordnet sind.

6. Kniegelenkendoprothese nach Anspruch 5, dadurch gekennzeichnet, daß der Femurteil (1) mit einer dem Kamm (70) entsprechend ausgebildeten bogenförmig zwischen den Gleitflächen (79, 80) der beiden Gleitkufen (77, 78) verlaufenden Unterkante (69) auf dem Kamm (70) in gestreckter Stellung der Prothese geführt ist.

7. Kniegelenkendoprothese nach einem der Ansprüche 3 bis 6, dadurch gekennzeichnet, daß die Querebene (99) gegenüber einer das Tibiaplateau (6) in seinem vorderen Teil (14) begrenzenden Vorderkante in Richtung auf das ihr gegenüberliegende Hinterteil (20) versetzt ist.

8. Kniegelenkendoprothese nach Anspruch 7, dadurch gekennzeichnet, daß die Querebene (99) um etwa ein Drittel einer sich in der Beugeebene (11) erstreckenden Längserstreckung der Unterseite (10) versetzt ist.

9. Kniegelenkendoprothese nach einem der Ansprüche 3 bis 8, dadurch gekennzeichnet, daß sich die dritte Rippe (16) bis etwa zum Hinterteil (20) des Tibiaplateaus (6) erstreckt.

10. Kniegelenkendoprothese nach einem der Ansprüche 1 bis 9, dadurch gekennzeichnet, daß eine sich durch den Bolzen (29) erstreckende Längsachse (31) in einem dem Hinterteil (20) zugewandten hinteren Drittel des Tibiaplateaus (6) quer zur Beugeebene (11) verläuft.

11. Kniegelenkendoprothese nach einem der Ansprüche 1 bis 10, dadurch gekennzeichnet, daß sich durch den Bolzen (29) eine horizontale Mittelebene (50) erstreckt, die einen Abstand von etwa 25 bis 30 cm vom Tibiaplateau (6) besitzt.

12. Kniegelenkendoprothese nach einem der Ansprüche 1 bis 12, dadurch gekennzeichnet, daß die Gleitkufen (77, 78) in Form eines Bogens (88) die Längsachse (31) in einem Abstand von etwa 20 bis 25 mm umgeben.

13. Kniegelenkendoprothese nach Anspruch 12, dadurch gekennzeichnet, daß der Bogen (88) den Bolzen (29) auf etwa der Hälfte seines Umfanges umgibt.

14. Kniegelenkendoprothese nach Anspruch 12 oder 13, dadurch gekennzeichnet, daß der Bogen (88) in einem einer vorderen Abschlußfläche (68) des Femurteils (1) zugewandten vorderen Teil (89) mit einem großen Krümmungsradius verläuft, der sich in Richtung auf einen hinteren Teil (21) verkleinert.

15. Kniegelenkendoprothese nach einem der Ansprüche 12 bis 14, dadurch gekennzeichnet daß der Bogen (88) mit seinem der vorderen Abschlußfläche (68) zugewandten vorderen Teil (89) in die vordere Abschlußfläche (68) des Gehäuses (61) einmündet, die dem vorderen Teil (14) des Tibiaplateaus (6) zugewandt ist.

16. Kniegelenkendoprothese nach Anspruch 15, dadurch gekennzeichnet daß die vordere Abschlußfläche (68) im gestreckten Zustand der Endoprothese gegenüber dem vorderen Teil (14) des Tibiaplateaus (6) in Richtung auf den Hinterteil (20) versetzt ist.

17. Kniegelenkendoprothese nach Anspruch 16, dadurch gekennzeichnet, daß in der vorderen Abschlußfläche (68) die den Kamm (70) im gestreckten Zustand der Endoprothese aufnehmende gewölbte Unterkante vorgesehen ist.

18. Kniegelenkendoprothese nach einem der Ansprüche 15 bis 17, dadurch gekennzeichnet, daß die vordere Abschlußfläche (68) im gestreckten Zustand der Endoprothese in einer Ebene verläuft, die schräg aufwärts vom Tibiaplateau (6) in Richtung auf den Femurschaft (58) verläuft und mit ihrer dem Tibiaplateau (6) zugewandten Unterkante (69) etwa im Bereich der Gleitflächen (79, 80) einmündet und in die auf der Unterseite (10) des Tibiaplateaus (6) der Tibiaschaft (9) in das Tibiaplateau (6) einmündet.

19. Kniegelenkendoprothese nach einem der Ansprüche 15 bis 18, dadurch gekennzeichnet, daß das Gehäuse (61) in Richtung auf den Femurschaft (58) von einer Deckplatte (56) abgeschlossen ist, die etwa senkrecht auf der vorderen Abschlußfläche (68) steht.

20. Kniegelenkendoprothese nach Anspruch 19, dadurch gekennzeichnet, daß der Femurschaft (58) in einen Bereich der Deckplatte (56) einmündet, in dem diese in die vordere Abschlußfläche (68) einmündet.

21. Kniegelenkendoprothese nach Anspruch 19 oder 20, dadurch gekennzeichnet, daß der Femurschaft (58) in gestreckter Stellung der Endoprothese in einer Querebene verläuft, die planparallel zur Querebene (99) des Tibiaschaftes (9) verläuft.

22. Kniegelenkendoprothese nach Anspruch 21, dadurch gekennzeichnet, daß die Querebene des Femurschaftes (58) in Richtung auf die Längsachse (31) gegenüber der Querebene (99) des Tibiaschaftes (9) versetzt ist.

23. Kniegelenkendoprothese nach Anspruch 22, dadurch gekennzeichnet, daß die Querebene des Femurschaftes (58) um etwa 0,5 mm bis 4 mm gegenüber der Querebene (99) des Tibiaschaftes (9) in Richtung auf die Längsachse (31) nach hinten versetzt ist.

24. Kniegelenkendoprothese nach einem der Ansprüche 20 bis 23, dadurch gekennzeichnet, daß der Femurschaft (58) in einer Neigung, die der Valgusstellung eines Femurknochens entspricht, gegenüber der Beugeebene (11) geneigt ist.

25. Kniegelenkendoprothese nach einem der Ansprüche 15 bis 24, dadurch gekennzeichnet, daß das Gehäuse (61) Seitenwände (62, 63) aufweist, in denen der Bolzen (29) gelagert ist und die etwa rechtwinklig in die vordere Abschlußfläche (68) einmünden.

26. Kniegelenkendoprothese nach Anspruch 25, dadurch gekennzeichnet, daß die Seitenwände (62, 63) eben sind und jeweils Bohrungen aufweisen, in denen Lagerschalen (39, 40) zur Lagerung des Bolzens (29) gehaltert sind.

27. Kniegelenkendoprothese nach einem der Ansprüche 4 bis 26, dadurch gekennzeichnet daß den Gleitflächen (79, 80) der Gleitkufen (77, 78) Aufnahmeflächen gegenüberliegen, die auf einer dem Femurschaft (58) zugewandten Oberseite (86, 87) der Gleitkufen (77, 78) ausgebildet sind.

28. Kniegelenkendoprothese nach Anspruch 27, dadurch gekennzeichnet, daß jede Aufnahmefläche sich über die gesamte Länge einer Gleitkufe (77, 78) erstreckt und etwa rechtwinklig in die Seitenwände (62, 63) des Gehäuses (61) einmünden.

29. Kniegelenkendoprothese nach Anspruch 28, dadurch gekennzeichnet, daß die Aufnahmefläche über eine Hohlkehle in die Seitenwände (62, 63) einmündet.

30. Kniegelenkendoprothese nach einem der Ansprüche 27 bis 29, dadurch gekennzeichnet, daß die Gleitflächen (79, 80) der Gleitkufen (77, 78) quer zu deren Längsrichtung ballig ausgebildet sind.

31. Kniegelenkendoprothese nach Anspruch 30, dadurch gekennzeichnet, daß die Gleitkufen (77, 78) je nach Ausbildung der Balligkeit eine Wandstärke von 1 bis 3 mm aufweisen.

32. Kniegelenkendoprothese nach Anspruch 30 oder 31, dadurch gekennzeichnet, daß der Hinterteil (20) des Tibiaplateaus (6) in seinem Mittelbereich eine sich in Richtung auf den Vorderteil (14) erstreckende Einbuchtung (94) aufweist, die beidseits von zwei Ausbuchtungen (95, 96) begrenzt wird, auf denen sich von den Gleitkufen (77, 78) beaufschlagbare Gleitflächen (75, 76) des Gleitaufsatzes (71) erstrecken, die im Bereich des vorderen Teils (14) hufeisenförmig durch einen den Kamm (70) ausbildenden Mittelteil (97) miteinander verbunden sind.

33. Kniegelenkendoprothese nach Anspruch 32, dadurch gekennzeichnet daß das Schwenkgelenk (4) mit seiner dem Hinterteil (20) zugewandten Hinterkante (47) oberhalb der Einbuchtung (94) angeordnet ist.

34. Kniegelenkendoprothese nach Anspruch 33, dadurch gekennzeichnet daß das Schwenkgelenk (4) mit seiner Längsachse (31) einen Abstand von etwa 25 bis 30 mm vom Tibiaplateau (6) hält.

35. Kniegelenkendoprothese nach einem der Ansprüche 1 bis 34, dadurch gekennzeichnet, daß das Gehäuse (61) zwischen seinen beiden Seitenwänden (62, 63) eine äußere Breite von etwa 30 bis 35 mm aufweist.

36. Kniegelenkendoprothese nach Anspruch 35, dadurch gekennzeichnet, daß die Seitenwände (62, 63) des Gehäuses (61) eine Dicke von etwa 5 bis 8 mm haben.

## Claims

1. A knee joint endoprosthesis system comprising a femur portion (1) and a tibia portion (2, 3) which are mounted pivotably about a hinge pin (29) of a hinge joint (4) connecting them together from an extended position into an angled position, have respective shanks (58, 9) which in the angled position include between them a bending angle (13) and in the extended position face in mutually opposite directions, of which a tibia shank (9) is connected to a tibia plate (6) at its upper end towards the femur portion (1) and a femur shank (58) is connected with its lower end towards the tibia portion (2, 3) to a housing (61), in whose walls (62, 63) the hinge pin (29) is mounted and to the lower ends of which, that are towards the tibia portion (2, 3), are fixed a respective slide portion (77, 78), characterised in that the knee joint endoprosthesis system comprises a rotary tibia portion (3), a hinge tibia portion (2) and a femur portion (1) which is mounted on the one hand rotationally movably relative to the associated rotary tibia portion (3) about a rotary axis (8) disposed perpendicularly to the tibia plate (6) and on the other hand rotationally immovably in a holder (21) fixedly connected to the hinge tibia portion (2) respectively, and the tibia portions (2, 3) relative to each other and the femur portion (1) with a respective tibia portion (2, 3) are of mutually equal outside dimensions.

2. A knee joint endoprosthesis according to claim 1 characterised in that the tibia shank (9) is fixed in a bending plane (11) including the bending angle (13) perpendicularly to an underside (10), which is towards it, of the tibia plate (6).

3. A knee joint endoprosthesis according to claim 1 or claim 2 characterised in that the tibia shank (9) is supported on the underside (10) of the tibia plate (6) with three ribs (15, 16, 17) of which two (15, 17) extend in a transverse plane (99) extending through the tibia shank (9) and oriented transversely with respect to the bending plane (11) and one (16) extends in the bending plane (11) in a direction towards a rear portion (20), which is included by the bending angle (13), of the tibia plate (6).

4. A knee joint endoprosthesis according to one of claims 1 to 3 characterised in that a sliding fitment portion (71) is fixed on the tibia plate (6), sliding surfaces (79, 80) of the slide portions (77, 78) facing towards the sliding fitment portion (71), on the two side portions (18, 19) of the tibia plate which are in mutually opposite relationship with respect to a central plane extending through the tibia shank (9) in plane-parallel relationship with the bending plane (11).

5. A knee joint endoprosthesis according to claim 4 characterised in that the side portions (18, 19) have slopes (73, 74) which rise towards a crest (70) and which are arranged in a front porton (14) of the tibia plate (6), which is opposite to the rear portion (20) of the tibia plate (6).

6. A knee joint endoprosthesis according to claim 5 characterised in that the femur portion (1) is guided in the extended position of the prosthesis on the crest (70) with a lower edge (69) which is of a configuration corresponding to the crest (70) and which extends arcuately between the sliding surfaces (79, 80) of the two slide portions (77, 78).

7. A knee joint endoprosthesis according to one of claims 3 to 6 characterised in that the transverse plane (99) is displaced relative to a front edge delimiting the tibia plate (6) in its front portion (14), towards the rear portion (20) disposed opposite same.

8. A knee joint endoprosthesis according to claim 7 characterised in that the transverse plane (99) is displaced by approximately one third of a longitudinal extent of the underside (10), extending in the bending plane (11).

9. A knee joint endoprosthesis according to one of claims 3 to 8 characterised in that the third rib (16) extends approximately as far as the rear portion (20) of the tibia plate (6).

10. A knee joint endoprosthesis according to one of claims 1 to 9 characterised in that a longitudinal axis (31) extending through the pin (29) extends in a rear third of the tibia plate (6) which is towards the rear portion (20), transversely with respect to the bending plane (11).

11. A knee joint endoprosthesis according to one of claims 1 to 10 characterised in that extending through the pin (29) is a horizontal central plane (50) which is at a spacing of about 25 to 30 cm from the tibia plate (6).

12. A knee joint endoprosthesis according to one of claims 1 to 12 characterised in that the slide portions (77, 78) extend in the form of an arc (88) around the longitudinal axis (31) at a spacing of about 20 to 25 mm.

13. A knee joint endoprosthesis according to claim 12 characterised in that the arc (88) extends around the pin (29) over approximately half its periphery.

14. A knee joint endoprosthesis according to claim 12 or claim 13 characterised in that in a front portion (89) which is towards the front terminal surface (68) of the femur portion (1) the arc (88) extends with a large radius of curvature which decreases in a direction towards a rear portion (21).

15. A knee joint endoprosthesis according to one of claims 12 to 14 characterised in that with its front portion (89) towards the front terminal surface (68) the arc (88) connects to the front terminal surface (68) of the housing (61), which is towards the front portion (14) of the tibia plate (6).

16. A knee joint endoprosthesis according to claim 15 characterised in that in the extended condition of the endoprosthesis the front terminal surface (68) is displaced relative to the front portion (14) of the tibia plate (6) in a direction towards the rear portion (20).

17. A knee joint endoprosthesis according to claim 16 characterised in that the curved lower edge which accommodates the crest (70) in the extended condition of the endoprosthesis is provided in the front terminal surface (68).

18. A knee joint endoprosthesis according to one of claims 15 to 17 characterised in that in the extended condition of the endoprosthesis the front terminal surface (68) extends in a plane which extends inclinedly upwardly from the tibia plate (6) towards the femur shank (58) and with its lower edge (69) towards the tibia plate (6) terminates approximately in the region of the sliding surfaces (79, 80), in which the tibia shank (9) connects to the tibia plate (6) on the underside (10) of the tibia plate (6).

19. A knee joint endoprosthesis according to one of claims 15 to 18 characterised in that the housing (61) is closed off towards the femur shank (58) by a cover plate (56) which is approximately perpendicular to the front terminal surface (68).

20. A knee joint endoprosthesis according to claim 19 characterised in that the femur shank (58) connects to a region of the cover plate (56) in which the latter joins the front terminal surface (68).

21. A knee joint endosprosthesis according to claim 19 or claim 20 characterised in that in the extended position of the endoprosthesis the femur shank (58) extends in a transverse plane which extends in plane-parallel relationship with the transverse plane (9) of the tibia shank (9).

22. A knee joint endoprosthesis according to claim 21 characterised in that the transverse plane of the femur shank (58) is displaced towards the longitudinal axis (31) with respect to the transverse plane (99) of the tibia shank (9).

23. A knee joint endosprosthesis according to claim 22 characterised in that the transverse plane of the femur shank (58) is displaced rearwardly in a direction towards the longitudinal axis (31) by about 0.5 mm to 4 mm with respect to the transverse plane (99) of the tibia shank (9).

24. A knee joint endoprosthesis according to one of claims 20 to 23 characterised in that the femur shank (58) is inclined relative to the bending plane (11) at an inclination corresponding to the valgus position of a femur bone.

25. A knee joint endoprosthesis according to one of claims 15 to 24 characterised in that the housing (61) has side walls (62, 63) in which the pin (29) is mounted and which connect approximately at a right angle to the front terminal surface (68).

26. A knee joint endoprosthesis according to claim 25 characterised in that the side walls (62, 63) are flat and have respective bores in which mounting shells (39, 40) for mounting the pin (29) are held.

27. A knee joint endoprosthesis according to one of claims 4 to 26 characterised in that disposed opposite the sliding surfaces (79, 80) of the slide portions (77, 78) are receiving surfaces which are provided on a top side (86, 87) of the slide portions (77, 78), which is towards the femur shank (58).

28. A knee joint endoprosthesis according to claim 27 characterised in that each receiving surface extends over the entire length of a slide portion (77, 78) and connects approximately at a right angle to the side walls (62, 63) of the housing (61).

29. A knee joint endoprosthesis according to claim 28 characterised in that the receiving surface connects to the side walls (62, 63) by way of a concave portion.

30. A knee joint endoprosthesis according to one of claims 27 to 29 characterised in that the sliding surfaces (79, 80) of the slide portions (77, 78) are of a spherical configuration transversely to the longitudinal direction thereof.

31. A knee joint endoprosthesis according to claim 30 characterised in that depending on the degree of spherical configuration involved the slide portions (77, 78) are of a wall thickness of 1 to 3 mm.

32. A knee joint endoprosthesis according to claim 30 or claim 31 characterised in that in its central region the rear portion (20) of the tibia plate (6) has a recess (94) which extends in a direction towards the front portion (14) and which is delimited on both sides by two raised portions (95, 96) on which extend sliding surfaces (75, 76) of the sliding fitment portion (71), which can be engaged by the slide portions (77, 78) and which are connected together in a horseshoe configuration in the region of the front portion (14) by a central portion (97) providing the crest (70).

33. A knee joint endoprosthesis according to claim 32 characterised in that the pivot joint (4) is arranged with its rear edge (47) which is towards the rear portion (20), above the recess (94).

34. A knee joint endoprosthesis according to claim 33 characterised in that the pivot joint (4) maintains with its longitudinal axis (31) a spacing of about 25 to 30 mm from the tibia plate (6).

35. A knee joint endoprosthesis according to one of claims 1 to 34 characterised in that the housing (61) is of an outside width of about 30 to 35 mm between its two side walls (62, 63).

36. A knee joint endoprosthesis according to claim 35 characterised in that the side walls (62, 63) of the housing (61) are of a thickness of about 5 to 8 mm.

## Revendications

1. Système d'endoprothèse d'articulation du genou comportant un élément fémoral (1) et un élément tibial (2, 3) qui sont montés avec possibilité de pivotement depuis une position étendue dans une position fléchie autour d'un axe de charnière (29) d'une articulation à charnière (4) reliant l'un à l'autre les deux éléments et comportent chacun des tiges (58, 9) qui, en position fléchie, forment entre elles un angle de flexion (13) et, en position étendue, sont orientées dans des directions opposées, parmi lesquelles une tige tibiale (9), à son extrémité supérieure côté élément fémoral (1), débouche dans un plateau tibial (6) et une tige fémorale (58), par son extrémité inférieure côté élément tibial (2, 3), débouche dans un corps (61), dans les parois (62, 63) duquel est logé l'axe de charnière (29) et aux extrémités inférieures tournées vers l'élément tibial (2, 3) duquel est fixé chaque fois un patin (77, 78), caractérisé par le fait que le système d'endoprothèse d'articulation du genou comprend un élément tibial (3) formant pivot, un élément tibial (2) formant charnière et un élément fémoral (1) qui d'une part est monté mobile en rotation par rapport à l'élément tibial (3) formant pivot autour d'un axe de rotation (8) perpendiculaire au plateau tibial (6) et d'autre part est monté fixe en rotation dans un support (21) lié rigidement à un élément tibial (2) formant charnière et par le fait que les éléments tibiaux (2, 3) entre eux d'une part et l'élément fémoral (1) et respectivement un élément tibial (2, 3) d'autre part présentent des dimensions extérieures identiques.

2. Endoprothèse d'articulation du genou selon la revendication 1, caractérisée par le fait que la tige tibiale (9), dans un plan de flexion (11) contenant l'angle de flexion (13), est fixée perpendiculairement sur une face inférieure du plateau tibial (6) tournée vers ladite tige tibiale.

3. Endoprothèse d'articulation du genou selon la revendication 1 ou 2, caractérisée par le fait que la tige tibiale (9) prend appui sur la face inférieure (10) du plateau tibial (6) par trois nervures (15, 16, 17) parmi lesquelles deux nervures (15, 17) s'étendent dans un plan transversal (99) passant par la tige tibiale (9) orienté transversalement au plan de flexion (11) et une nervure (16) s'étend dans le plan de flexion (11) en direction d'une partie postérieure (20) du plateau tibial (6) englobée dans l'angle de flexion (13).

4. Endoprothèse d'articulation du genou selon l'une des revendications 1 à 3, caractérisée par le fait qu'une garniture de glissement (71) est fixée sur le plateau tibial (6), garniture vers les deux parties latérales (18, 19) disposées en vis-à-vis par rapport à un plan médian parallèle au plan de flexion (11) passant par la tige tibiale (9) de laquelle sont tournées des surfaces de glissement (79, 80) des patins (77, 78).

5. Endoprothèse d'articulation du genou selon la revendication 4, caractérisée par le fait que les parties latérales (18, 19) présentent des plans inclinés (73,74) qui s'élèvent en direction d'une arête (70) et sont disposés dans une partie antérieure (14) du plateau tibial (6) en vis-à-vis de la partie postérieure (20) du plateau tibial (6).

6. Endoprothèse d'articulation du genou selon la revendication 5, caractérisée par le fait que l'élément fémoral (1), dans la position étendue de la prothèse, est guidé sur l'arête (70) par un bord inférieur (69) adapté à ladite arête (70), qui s'étend en arc de cercle entre les surfaces de glissement (79, 80) des deux patins (77, 78).

7. Endoprothèse d'articulation du genou selon l'une des revendications 3 à 6, caractérisée par le fait que le plan transversal (99) est décalé en direction de la partie postérieure (20) par rapport à un bord antérieur qui délimite le plateau tibial (6) dans sa partie antérieure (14).

8. Endoprothèse d'articulation du genou selon la revendication 7, caractérisée par le fait que le plan transversal (99) est décalé d'environ un tiers de la longueur de la face inférieure (10) située dans le plan de flexion (11).

9. Endoprothèse d'articulation du genou selon l'une des revendications 3 à 8, caractérisée par le fait que la troisième nervure (16) s'étend sensiblement jusqu'à la partie postérieure (20) du plateau tibial (6).

10. Endoprothèse d'articulation du genou selon l'une des revendications 1 à 9, caractérisée par le fait qu'un axe longitudinal (31) passant par l'axe (29) s'étend transversalement au plan de flexion (11) dans un tiers arrière du plateau tibial (6), côté partie postérieure (20).

11. Endoprothèse d'articulation du genou selon l'une des revendications 1 à 10, caractérisée par le fait qu'un plan médian (50) horizontal passe par l'axe (29), lequel plan médian est situé à une distance d'environ 25 à 30 cm du plateau tibial (6).

12. Endoprothèse d'articulation du genou selon l'une des revendications 1 à 11, caractérisée par le fait que les patins (77, 78) entourent en formant un arc (88) l'axe longitudinal (31) à une distance d'environ 20 à 25 cm.

13. Endoprothèse d'articulation du genou selon la revendication 12, caractérisée par le fait que l'arc (88) entoure l'axe (29) sur environ la moitié de son périmètre.

14. Endoprothèse d'articulation du genou selon la revendication 12 ou 14, caractérisée par le fait que l'arc (88), dans une partie antérieure (89) tournée vers une surface de fermeture (68) antérieure de l'élément fémoral (1), présente un grand rayon de courbure qui diminue en direction d'une partie postérieure (21).

15. Endoprothèse d'articulation du genou selon l'une des revendications 12 à 14, caractérisée par le fait que l'arc (88) se raccorde par sa partie antérieure (89) tournée vers la surface de fermeture (68) antérieure à la surface de fermeture (68) antérieure du corps (61) tournée vers la partie antérieure (14) du plateau tibial (6).

16. Endoprothèse d'articulation du genou selon la revendication 15, caractérisée par le fait que la surface de fermeture (68) antérieure, dans la position étendue de l'endoprothèse, est décalée en direction de la partie postérieure (20) par rapport à la partie antérieure (14) du plateau tibial (6).

17. Endoprothèse d'articulation du genou selon la revendication 16, caractérisée par le fait que le bord inférieur arqué qui reçoit l'arête (70) dans la position étendue de l'endoprothèse est prévu dans la surface d'extrémité (68) antérieure.

18. Endoprothèse d'articulation du genou selon l'une des revendications 15 à 17, caractérisée par le fait que la surface de fermeture (68) antérieure, dans la position étendue de l'endoprothèse, est située dans un plan qui s'étend en biais, vers le haut, du plateau tibial (6) vers la tige fémorale (58) et, par son bord (69) inférieur tourné vers le plateau tibial (6), débouche sensiblement dans la région des surfaces de glissement (79, 80) et dans le plateau tibial (6) la surface sur la face inférieure (10) du plateau tibial (6) tournée vers la tige tibiale (9).

19. Endoprothèse d'articulation du genou selon l'une des revendications 15 à 19, caractérisée par le fait que le corps (61), côté tige fémorale (58), est fermé par une plaque de couverture (56) qui est sensiblement perpendiculaire à la surface de fermeture (68) antérieure.

20. Endoprothèse d'articulation du genou selon la revendication 19, caractérisée par le fait que la tige fémorale (58) se raccorde à une région de la plaque de couverture (56) dans laquelle ladite plaque se raccorde à la surface de fermeture (68) antérieure.

21. Endoprothèse d'articulation du genou selon la des revendication 19 ou 20, caractérisée par le fait que la tige fémorale (58), dans la position étendue de l'endoprothèse, est située dans un plan transversal qui s'étend parallèlement au plan transversal (99) de la tige tibiale (9).

22. Endoprothèse d'articulation du genou selon la revendication 21, caractérisée par le fait que le plan transversal de la tige fémorale (58) est décalé en direction de l'axe longitudinal (31) par rapport au plan transversal (99) de la tige tibiale (9).

23. Endoprothèse d'articulation du genou selon la revendication 22, caractérisée par le fait que le plan transversal de la tige fémorale (58) est décalé d'environ 0,5 mm à 4 mm vers l'arrière en direction de l'axe longitudinal (31), par rapport au plan transversal (99) de la tige tibiale (9).

24. Endoprothèse d'articulation du genou selon l'une des revendications 20 à 23, caractérisée par le fait que la tige fémorale (58) est inclinée par rapport au plan de flexion (11) avec une inclinaison qui correspond à la position de valgus d'un os fémoral.

25. Endoprothèse d'articulation du genou selon l'une des revendications 15 à 24, caractérisée par le fait que le corps (61) comporte des parois latérales (62, 63) dans lesquelles l'axe (29) est logé et qui se raccordent sensiblement à angle droit avec la surface de fermeture (68) antérieure.

26. Endoprothèse d'articulation du genou selon la revendication 25, caractérisée par le fait que les parois latérales (62, 63) sont planes et présentent chacune des trous pour le montage de l'axe (29), dans lesquels sont insérées des douilles de paliers (39, 40).

27. Endoprothèse d'articulation du genou selon l'une des revendications 4 à 26, caractérisée par le fait que des surfaces réceptrices sont disposées en vis-à-vis des surfaces de glissement (79, 80) des patins (77, 78), lesquelles surfaces réceptrice sont agencées sur une face supérieure (86, 87) des patins (77, 78) tournée vers la tige fémorale (58).

28. Endoprothèse d'articulation du genou selon la revendication 27, caractérisée par le fait que chaque surface réceptrice s'étend sur toute la longueur d'un patin (77, 78) et se raccorde sensiblement à angle droit avec les parois latérales (62, 63) du corps (61).

29. Endoprothèse d'articulation du genou selon la revendication 28, caractérisée par le fait que la surface de réception débouche dans les parois latérales (62, 63) par l'intermédiaire d'un congé.

30. Endoprothèse d'articulation du genou selon l'une des revendications 27 à 29, caractérisée par le fait que les surfaces de glissement (79, 80) des patins (77, 78) sont bombées dans la direction transversale à leur longueur.

31. Endoprothèse d'articulation du genou selon la revendication 30, caractérisée par le fait que les patins (77, 78), selon le bombé, ont une épaisseur de parois allant de 1 mm à 3 mm.

32. Endoprothèse d'articulation du genou selon la revendication 30 ou 31, caractérisée par le fait que la partie postérieure (20) du plateau tibial (6), dans sa région médiane, comporte une cavité (94) qui s'étend en direction de la partie antérieure (14), est délimitée des deux côtés par deux renflements (95, 96) sur lesquels s'étendent des surfaces de glissement (75, 76) de la garniture de glissement (71) en prise avec les patins (77, 78), lesquelles surfaces de glissement, dans la région de la partie antérieure, sont reliées en une forme de fer à cheval par une partie médiane qui forme l'arête (70).

33. Endoprothèse d'articulation du genou selon la revendication 32, caractérisée par le fait que le bord postérieur (47) de l'articulation de pivotement (4) tourné vers la partie postérieure (20) est disposé au-dessus de la cavité (94).

34. Endoprothèse d'articulation du genou selon la revendication 33, caractérisée par le fait que l'axe longitudinal de l'articulation de pivotement (4) est disposé à une distance d'environ 25 à 30 mm du plateau tibial (6).

35. Endoprothèse d'articulation du genou selon l'une des revendications 1 à 34, caractérisée par le fait que le corps (61), entre ses deux parois latérales (62, 63) a une largeur extérieure d'environ 30 à 35 mm.

36. Endoprothèse d'articulation du genou selon la revendication 35, caractérisée par le fait que les parois latérales (62, 63) du corps (61) ont une épaisseur d'environ 5 à 8 mm.
